# FASCICULE DE BREVET EUROPEEN

(11) **EP 3 397 378 B1**
(45) Date de publication et mention de la délivrance du brevet: **15.12.2021**
(21) Numéro de dépôt: 16829272.0
(22) Date de dépôt: 22.12.2016
(51) Int. Cl.: B01J 13/10, A01N 25/28, A61K 8/11, A61K 9/50, C11D 3/50

(54) **MICROCAPSULE COMPRENANT UNE MEMBRANE ISSUE D'UNE MICROENCAPSULATION PAR COACERVATION COMPLEXE, ET PROCÉDÉ D'OBTENTION**
MIKROKAPSEL MIT EINER MEMBRAN, HERGESTELLT DURCH MIKROVERKAPSELUNG UNTER VERWENDUNG VON KOMPLEXER KOAZERVIERUNG UND HERSTELLUNGSVERFAHREN
MICROCAPSULE COMPRISING A MEMBRANE OBTAINED BY MICROENCAPSULATION USING COMPLEX COACERVATION, AND OBTENTION METHOD

(30) Priorité: 28.12.2015 FR 1563371
(43) Date de publication de la demande: 07.11.2018
(73) Titulaire: Capsulae, 17000 La Rochelle (FR)
(72) Inventeur: ONGMAYEB, Gisèle, 44700 Orvault (FR); COSTARD, Magali, 44100 Nantes (FR)
(74) Mandataire: Jacobacci Coralis Harle
(86) Numéro de dépôt international: PCT/FR2016/053628
(87) Numéro de publication internationale: WO 2017/115034

(56) Documents cités:
- WO-A1-00/69420
- FR-A1- 2 340 087
- DATABASE WPI Week 201277 Thomson Scientific, London, GB; AN 2012-P42181 XP002761020, & CN 102 641 703 A (BOE TECHNOLOGY GROUP CO LTD) 22 août 2012 (2012-08-22)
- MWANGI WILLIAM WACHIRA ET AL: "Facile method for forming ionically cross-linked chitosan microcapsules from Pickering emulsion templates", FOOD HYDROCOLLOIDS, vol. 55, 4 novembre 2015 (2015-11-04), pages 26-33, XP029367551, ISSN: 0268-005X, DOI: 10.1016/J.FOODHYD.2015.10.022
- PARK SANGPHIL ET AL: "Magnetic nanoparticle-embedded PCM nanocapsules based on paraffin core and polyurea shell", COLLOIDS AND SURFACES. A, PHYSICACHEMICAL AND ENGINEERING ASPECTS, vol. 450, 12 mars 2014 (2014-03-12), pages 46-51, XP028845980, ISSN: 0927-7757, DOI: 10.1016/J.COLSURFA.2014.03.005

## Description

### DOMAINE TECHNIQUE AUQUEL SE RAPPORTE L'INVENTION

La présente invention concerne de manière générale le domaine de la microencapsulation.

Elle concerne plus particulièrement des microcapsules comportant un système d'enveloppe comprenant une membrane obtenue par coacervation complexe.

### ARRIERE-PLAN TECHNOLOGIQUE

La microencapsulation est aujourd'hui couramment utilisée pour la protection et pour le contrôle de la libération de principes actifs.

Cette technologie consiste à enfermer une substance solide, liquide ou pâteuse, dans un système d'enveloppe sphérique solide (généralement en polymères). La taille des microparticules obtenues varie généralement du micromètre au millimètre.

Les microcapsules sont en particulier utilisées dans les secteurs industriels comme l'agriculture, les produits sanitaires, la santé, la cosmétique, la chimie des détergents ou encore le textile.

En pratique, le système d'enveloppe est généralement formé par polymérisation, réticulation interfaciale ou coacervation complexe.

En raison de sa simplicité et de sa faible toxicité, la microencapsulation dite par « coacervation complexe » est souvent utilisée pour préparer des microcapsules destinées à la nutrition humaine ou animale, à la santé ou à la cosmétique.

La coacervation complexe repose sur le phénomène de désolvatation de macromolécules (polymères) de charges opposées, aboutissant à la formation de deux phases non miscibles, cela à partir de solutions aqueuses colloïdales initialement homogènes.

Les deux phases obtenues par coacervation complexe sont :
- le coacervat, riche en macromolécules et appauvri en eau, qui résulte de la formation de complexes entre les macromolécules chargées positivement et celles chargées négativement, et
- le surnageant, pauvre en macromolécules et riche en eau.

Pour cela, l'encapsulation d'une huile par coacervation complexe consiste à émulsionner l'huile dans une solution aqueuse de deux polymères.

La coacervation est ensuite induite habituellement par ajustement du pH (généralement une acidification), de sorte que les deux polymères comportent des charges opposées pour former des complexes polymères. Les complexes polymères formés s'adsorbent sur les gouttelettes d'huile et les isolent ainsi du milieu extérieur.

La paroi ainsi formée peut ensuite être durcie par refroidissement du milieu et réticulée par l'action d'un agent de réticulation.

Dans un tel procédé de microencapsulation par coacervation complexe, la formation préalable de l'émulsion constitue une étape cruciale.

Cette étape requiert généralement l'utilisation de tensioactifs chimiques spécifiques ou de polymères (gélatine, protéines, etc.) qui s'adsorbent à l'interface des gouttelettes générées par un système d'agitation, et qui permettent de diminuer et de fixer la taille des gouttelettes tout en les stabilisant.

La plupart des tensioactifs actuellement utilisés sont cependant de nature chimique, ce qui limite leur utilisation dans les domaines de la cosmétique et de l'alimentation notamment.

En outre, les tensioactifs n'améliorent ni la stabilité finale des microcapsules, ni leur résistance mécanique.

Il reste donc nécessaire de développer de nouvelles microcapsules, répondant notamment aux normes agroalimentaires et/ou cosmétiques, qui présenteraient en outre une stabilité thermique et une résistance mécanique optimales. CN 102 641 703 A divulgue un procédé pour la fabrication des microcapsules, caractérisé en ce qu'il comprend l'obtention d'une émulsion de Pickering et la microencapsulation par coacervation utilisant de la gélatine.

### OBJET DE L'INVENTION

Afin de répondre à l'objectif précité, la présente invention propose des microcapsules comprenant un contenu hydrophobe entouré par un système d'enveloppe qui comprend une membrane comprenant une combinaison d'au moins deux polyélectrolytes hydrosolubles complémentaires choisis parmi les polyélectrolytes hydrosolubles aptes à générer une microencapsulation par coacervation complexe.

Selon l'invention, ledit système d'enveloppe comprend également des particules solides choisies parmi les particules solides aptes à stabiliser (dit encore « à former » ou « à obtenir ») une émulsion de Pickering.

Le contenu hydrophobe contient au moins un principe actif, lequel au moins un principe actif est sensible aux rayonnements ultraviolets.

De telles microcapsules ont l'intérêt de répondre aux critères agroalimentaires et/ou cosmétiques.

Elles présentent également une stabilité thermique et une résistance mécanique optimales.

Ces microcapsules permettent encore :
- une protection à l'encontre des phénomènes d'oxydation,
- une photostabilité, et une protection à l'encontre des rayonnements ultraviolets, pour le produit encapsulé, et
- un ralentissement de la libération d'un principe actif dans un milieu donné.

D'autres caractéristiques non limitatives et avantageuses des microcapsules conformes à l'invention, prises individuellement ou selon toutes les combinaisons techniquement possibles, sont les suivantes :
- Les particules solides peuvent être choisies parmi les particules inorganiques, par exemple les argiles, les silices colloïdales, les latex et/ou les particules organiques, par exemple les amidons, les chitosan, les chitines, les protéines ;
- Les polyélectrolytes hydrosolubles sont de préférence d'origine naturelle ;
- Ladite combinaison d'au moins deux polyélectrolytes hydrosolubles complémentaires comprend au moins deux polyélectrolytes hydrosolubles ayant des charges opposées, à savoir au moins un premier polyélectrolyte hydrosoluble ayant des charges positives, de préférence une protéine, et au moins un second polyélectrolyte hydrosoluble ayant des charges négatives, de préférence un polysaccharide ;
- Ledit au moins un premier polyélectrolyte hydrosoluble chargé positivement peut être choisi parmi les gélatines, les protéines (par exemple les protéines de pois, les protéines de lait), le chitosane ;
- Ledit au moins un second polyélectrolyte hydrosoluble chargé négativement peut être choisi parmi les gommes arabiques, les carboxyméthylcelluloses, les pectines, les alginates ;
- Les polyélectrolytes hydrosolubles sont de préférence dans un état réticulé ;
- Le contenu hydrophobe de la microcapsule peut être choisi parmi les huiles, notamment les huiles végétales, les huiles pharmaceutiques (notamment le miglyol - marque déposée), les solvants organiques ou un matériau à changement de phase ;
- Le contenu hydrophobe contient au moins un principe actif, sensible aux rayonnements ultraviolets (c'est-à-dire présentant une perte d'activité supérieure à 20% lorsqu'il est soumis aux rayonnements ultraviolets) ;
- Le système d'enveloppe de la microcapsule selon l'invention est dépourvu d'agents tensioactifs synthétiques ;
- La microcapsule selon l'invention a une taille inférieure à 800 µm, de préférence de 1 à 100 µm, de préférence encore de 1 à 50 µm.

L'invention propose également des compositions contenant des microcapsules selon l'invention.

L'invention propose encore un procédé pour la fabrication de microcapsules caractérisé en ce qu'il comprend :
- l'obtention de gouttelettes stabilisées par des particules solides choisies parmi les particules solides aptes à stabiliser une émulsion de Pickering, puis
- la microencapsulation par coacervation complexe desdites gouttelettes par une combinaison d'au moins deux polyélectrolytes hydrosolubles complémentaires choisis parmi les polyélectrolytes hydrosolubles aptes à générer une microencapsulation par coacervation complexe.

Dans une première variante, le procédé comprend les étapes suivantes :
(a) le mélange d'une solution lipidique et d'une solution aqueuse contenant des particules solides, d'une part, aptes à stabiliser une émulsion de Pickering et, d'autre part, à former l'un desdits deux polyélectrolytes hydrosolubles, pour former une émulsion de Pickering,
(b) le mélange de ladite émulsion de Pickering issue de l'étape (a) avec au moins un second polyélectrolyte hydrosoluble complémentaire, ledit second polyélectrolyte hydrosoluble étant avantageusement en solution aqueuse.
(c) la coacervation complexe pour obtenir lesdites microcapsules.

Dans une seconde variante, le procédé est caractérisé en ce qu'il comprend les étapes suivantes :
(a) le mélange d'une solution lipidique et d'une solution aqueuse contenant des particules solides aptes à former une émulsion de Pickering, pour la formation d'une émulsion de Pickering,
(b) le mélange de ladite émulsion de Pickering issue de l'étape (a) et de ladite combinaison de polyélectrolytes hydrosolubles complémentaires, les premier et second polyélectrolytes hydrosolubles étant ajoutés de façon simultanée ou successive à ladite émulsion de Pickering, et
(c) la coacervation complexe pour obtenir lesdites microcapsules.

Dans une troisième variante, le procédé est caractérisé en ce qu'il comprend les étapes suivantes :
(a) le mélange d'une solution lipidique et d'une solution aqueuse contenant des particules solides aptes à former une émulsion de Pickering et ladite combinaison de polyélectrolytes hydrosolubles complémentaires, pour former une émulsion de Pickering, et
(b) la coacervation complexe de mélange issu de l'étape (a).

D'autres caractéristiques non limitatives et avantageuses du procédé conforme à l'invention, prises individuellement ou selon toutes les combinaisons techniquement possibles, sont les suivantes :
- l'étape (b) du procédé de l'invention pour les premier et second modes comprend :
   (b1) le mélange de ladite émulsion de Pickering avec une première solution aqueuse comprenant ledit premier polyélectrolyte hydrosoluble, avantageusement le polyélectrolyte hydrosoluble cationique, pour obtenir un premier mélange,
   (b2) le mélange dudit premier mélange avec une seconde solution aqueuse contenant ledit second polyélectrolyte hydrosoluble complémentaire, avantageusement ledit polyélectrolyte hydrosoluble anionique, pour obtenir un second mélange ;
- L'étape de coacervation complexe du procédé comprend :
   (i) la modification du pH du mélange, le cas échéant une acidification jusqu'à atteindre un pH inférieur au point isoélectrique d'une protéine formant l'un desdits polyélectrolytes hydrosolubles, et éventuellement
   (ii) un refroidissement dudit mélange issu de l'étape (i) ;
- L'étape de coacervation complexe est de préférence suivie d'une étape de réticulation desdits polyélectrolytes hydrosolubles complémentaires par le biais d'au moins un agent réticulant ; l'agent réticulant est avantageusement choisi parmi les agents réticulant naturels, notamment la transglutaminase ou la génépine.

L'invention concerne aussi l'utilisation de microcapsules selon l'invention pour la protection, à l'égard des rayonnements ultraviolets, d'un principe actif sensible auxdits rayonnements ultraviolets

### DESCRIPTION DETAILLEE DE L'INVENTION

La description qui va suivre, en regard des résultats expérimentaux donnés à titre d'exemples non limitatifs, fera bien comprendre en quoi consiste l'invention et comment elle peut être réalisée.

Sauf précision contraire, il est entendu selon la présente invention, que les différents modes de réalisation décrits ci-après peuvent être combinés entre eux.

Dans les figures annexées :

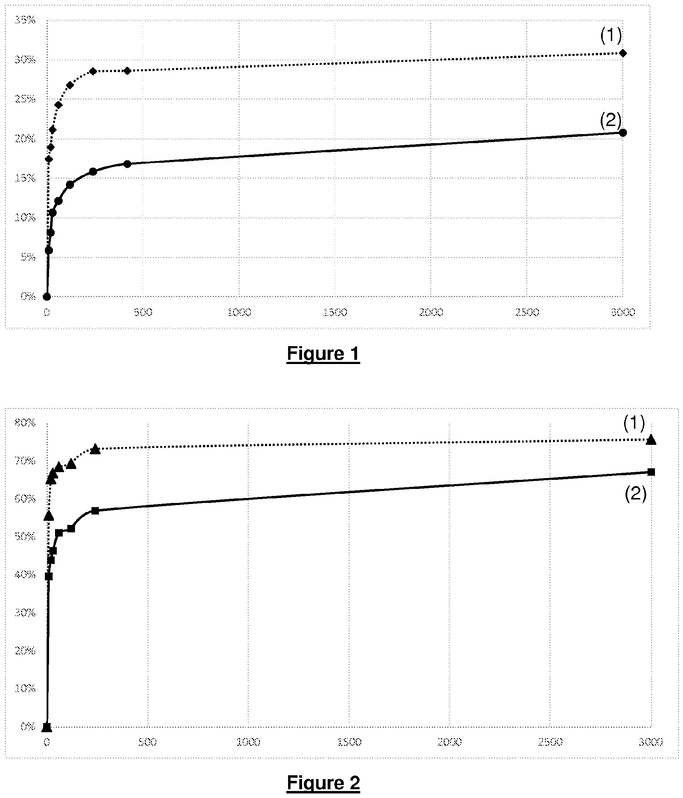

- la figure 1 illustre la libération, en fonction du temps, de paprika encapsulé dans (1) des microcapsules réalisées de polymères seulement (sans présence de particules solides) et (2) des microcapsules réalisées avec une combinaison de particules solides et de polymères - Légende : relargage cumulé (%) en fonction du temps en minutes ;
- la figure 2 illustre la libération, en fonction du temps et après stockage à 50°C pendant une semaine, de paprika encapsulé dans (1) des microcapsules réalisées de polymères seulement (sans présence de particules solides) et (2) des microcapsules réalisées avec une combinaison de particules solides et de polymères - Légende : relargage cumulé (%) en fonction du temps en minutes.

### Microcapsules selon l'invention

L'invention se rapporte à des microcapsules comprenant un contenu hydrophobe, entouré par un système d'enveloppe qui comprend :
- une membrane comprenant une combinaison d'au moins deux polyélectrolytes hydrosolubles choisis parmi les couples de polyélectrolytes hydrosolubles aptes à générer une microencapsulation par coacervation complexe, et
- des particules solides choisies parmi les particules solides aptes à stabiliser une émulsion de Pickering.

Selon l'invention, par « microencapsulation », on entend en particulier le procédé par lequel on enferme un produit (solide, liquide ou pâteux), dans des microcapsules.

Toujours selon l'invention, par « microcapsule », on entend en particulier un élément du type « coacervat », c'est-à-dire une petite gouttelette sphéroïdale de particules colloïdales en suspension, dont la cohérence par rapport au liquide environnant est assurée par les forces hydrophobes du contenu. Cette microcapsule selon l'invention est ainsi avantageusement du type vésiculaire.

La microcapsule selon l'invention constitue ainsi une particule solide constituée d'un système d'enveloppe solide entourant un contenu hydrophobe (liquide, solide ou pâteux), ledit système d'enveloppe étant obtenu à partir d'un procédé comprenant au moins une technique de coacervation complexe.

Par « système d'enveloppe », on entend en particulier un ensemble de composés entourant le contenu hydrophobe, qui isole et protège ce contenu hydrophobe à l'égard du milieu extérieur, et/ou qui permet une maîtrise de sa libération dans un environnement choisi.

En l'espèce, le système d'enveloppe des microcapsules selon l'invention combine - une membrane issue d'une coacervation complexe (dite encore « membrane de coacervat ») et - des particules solides pour émulsion de Pickering.

### Membrane issue d'une coacervation complexe

Le terme « membrane » signifie, selon l'invention, une paroi sensiblement continue de matériaux polyélectrolytes hydrosolubles, réticulés ou non, et formés autour du contenu hydrophobe (solide ou liquide) encapsulé.

Selon l'invention, cette membrane est issue d'une technique de coacervation complexe, c'est-à-dire une désolvatation simultanée de deux polyélectrolytes hydrosolubles portant des charges opposées, qui est avantageusement provoquée par une modification de pH du milieu aqueux.

La membrane de coacervation complexe comprend ainsi une combinaison d'au moins deux polyélectrolytes hydrosolubles complémentaires choisis parmi les couples de polyélectrolytes hydrosolubles aptes à générer une telle microencapsulation par coacervation complexe.

Par « polyélectrolytes hydrosolubles complémentaires », on entend ainsi au moins des premier et second polyélectrolytes hydrosolubles qui sont aptes à coopérer pour générer un phénomène de microencapsulation par coacervation complexe.

Pour cela, la combinaison de polyélectrolytes hydrosolubles selon l'invention comprend au moins deux polyélectrolytes hydrosolubles ayant des charges opposées, notamment pour une valeur de pH déterminée (désigné ici « pH de coacervation » dans un souci de simplification).

La combinaison de polyélectrolytes hydrosolubles selon l'invention comprend ainsi (le cas échéant à la valeur de pH de coacervation) :
- au moins un polyélectrolyte hydrosoluble cationique, chargé positivement, et
- au moins un polyélectrolyte hydrosoluble anionique, chargé négativement.

Par « polyélectrolytes hydrosolubles », on entend des polymères dont les unités répétées portent un groupe électrolyte. Ces groupes électrolytes sont destinés à se dissocier en milieux aqueux, formant ainsi des polymères chargés soit positivement (cationiques) soit négativement (anioniques).

Des polyélectrolytes hydrosolubles convenant à l'invention peuvent être choisis parmi les protéines, les polysaccharides, les polyterpènes, les polyphosphates, les latex, les polymères thermoplastiques, comme les polyhydroxyalcanoates, les polyméthacrylates, les polymères d'alcools polyvinylique, ou encore les polymères styréniques.

De préférence, les polyélectrolytes hydrosolubles sont choisis parmi les biopolymères.

Les biopolymères sont des polymères d'origine naturelle, c'est-à-dire issus de la biomasse (produits par des êtres vivants végétaux, algaux, animaux, fongiques, etc.).

De tels polyélectrolytes hydrosolubles peuvent alors être choisis parmi les protéines et leurs dérivés (incluant les polypeptides), les polysaccharides, les polyterpènes, les polynucléotides ou les polyhydroxyalcanoates.

Ces biopolymères peuvent être naturels ou semi-naturels (c.à.d. des polymères d'origine naturelle mais ayant subi des modifications chimiques après purification). De préférence, seuls des biopolymères naturels sont utilisés.

L'utilisation de tels biopolymères est particulièrement appropriée dans les domaines de la santé, de la nutrition ou de la cosmétique, pour lesquels l'utilisation de composants chimiques synthétiques est restreinte.

Des biopolymères particulièrement adaptés à l'invention sont typiquement les protéines et les polysaccharides, ainsi que leurs dérivés.

Parmi les protéines et leurs dérivés utilisables en tant que polyélectrolytes selon l'invention, on peut citer, de façon non limitative, les protéines du lait comme les protéines du lactosérum (notamment la béta lactoglobuline) et la caséine, l'albumine, la gélatine, les protéines végétales, le collagène, et leurs dérivés.

Les protéines végétales utilisables en tant que polyélectrolytes selon l'invention peuvent être extraites de végétaux choisis dans le groupe comprenant : le lupin (genre *Lupinus*), le soja (genre *Glycine*), le pois (genre *Pisum*), le pois chiche (*Cicer*), la luzerne (*Medicago*), les féveroles (*Vicia*), les lentilles (*Lens*), le haricot (*Phaseolus*), le colza (*Brassica*), le tournesol (*Helianthus*) et des céréales comme le blé, le maïs, l'orge, le malt et l'avoine.

Les protéines de soja (telle que la protéine commercialisée sous le nom de SUPRO^{®} 670), la gliadine, le gluten de blé et les protéines de pois (notamment la viciline ou la protéine commercialisée sous le nom de PISANE^{®}) sont des protéines végétales particulièrement avantageuses.

Parmi les polysaccharides utilisables en tant que polyélectrolytes selon l'invention, on peut citer, de façon non limitative, les glycosaminoglycanes, comme les hyaluronates, le chondroïtine sulfate ou l'héparine ; la gomme arabique (ou gomme d'acacia) ; la gomme de Karaja ; la gomme de tragacanth ; la gomme xanthane ; l'agar agar ; les alginates ; le dextran ; l'amidon ; la cellulose ; les carraghénanes ; l'amylopectine ; le chitosan (ou chitosane) ; la pectine ; et/ou leurs dérivés respectifs.

Parmi les dérivés de cellulose, on inclut notamment les carboxyméthylcelluloses (CMC), les méthylcelluloses, l'hydroxypropylcellulose (HPC), les nitrocelluloses et la gomme de cellulose.

En l'espèce, le polyélectrolyte anionique est avantageusement choisi parmi ceux classiquement utilisés par l'homme du métier : les alginates (notamment l'alginate de sodium), la gomme arabique (ou gomme d'acacia), les polyphosphates, les carboxyméthylcelluloses de sodium, les carraghénanes, la gomme xanthane, la pectine, la chondroïtine sulfate, la gomme Karaja, la gomme tragacanth, l'héparine ou le gluten de blé.

De son côté, le polyélectrolyte cationique est avantageusement un de ceux classiquement utilisés par l'homme du métier : les latex possédant un ammonium quaternaire, le chitosane, la gélatine, l'albumine, le collagène, la caséine, et les protéines de pois, les protéines de soja ou les protéines du lactosérum.

Par « gélatine », on englobe notamment les gélatines issues de porc, de bœuf et de poisson.

On pourra utiliser notamment de la gélatine :
- de type A, produite à partir d'os et couenne de porc, dont le point isoélectrique est supérieur à 6,5, ou
- de type B, produite à partir des os et peaux de bovins, dont le point isoélectrique est d'environ 5.

De préférence, on utilisera une gélatine de type A.

Les exemples ci-dessus de protéines et de polysaccharides peuvent être associés entre eux pour former la combinaison de l'invention, dans la mesure où ils sont de charges opposées.

Ainsi, ladite combinaison est avantageusement formée d'au moins un couple choisi parmi les couples suivants : protéine / protéine, protéine / polysaccharide ou polysaccharide / polysaccharide.

De préférence encore, la combinaison de polyélectrolytes selon l'invention comprend au moins une protéine et au moins un polysaccharide qui ont respectivement des charges opposées à une valeur de pH de coacervation.

De manière générale, l'un au moins des polyélectrolytes hydrosolubles est avantageusement choisi parmi les composés ayant un caractère amphotère, en particulier les protéines ayant l'intérêt d'avoir une charge globale inversable en fonction de la valeur du pH.

A cet égard, une protéine présente un point isoélectrique (pHi ou pl), c'est-à-dire une valeur de pH pour laquelle la charge globale de cette molécule est nulle ou, autrement dit, le pH pour lequel la molécule est électriquement neutre (forme zwitterionique ou ion mixte).

Si le pH est inférieur au pl, la charge globale de la protéine est positive ; si le pH est supérieur au pl, la charge globale de la molécule est négative.

Selon un mode de réalisation préféré, le polyélectrolyte hydrosoluble cationique est choisi parmi les protéines et le polyélectrolyte hydrosoluble anionique est choisi parmi les polysaccharides.

La protéine est avantageusement choisie avec un pl supérieur au pH de coacervation. Par exemple, cette protéine est choisie parmi les protéines ayant un pl allant de 4 à 8.

En pratique, cette protéine est destinée à présenter - une charge globale négative lors de son mélange avec le polyélectrolyte hydrosoluble anionique et - une charge globale positive lors du phénomène de coacervation complexe. Dans un souci de simplification, la protéine sera alors désignée sous l'appellation de « polyélectrolyte hydrosoluble cationique », complémentaire du polyélectrolyte hydrosoluble anionique.

Dans un souci de simplification, la protéine est désignée en tant que polyélectrolyte hydrosoluble cationique

A titre d'exemple, la combinaison de polyélectrolytes hydrosolubles peut être choisie parmi les combinaisons suivantes : gomme d'acacia / gélatine, pectine / caséine, pectine / gélatine, alginate / gélatine, alginate / chitosan, gomme xanthane / chitosan, pectine / protéine de soja, gomme d'acacia / protéine de soja, gomme d'acacia / protéine du lactosérum, pectine / protéine du lactosérum, héparine / gélatine, hydroxypropylméthylcellulose (HPMC) / sodium dodécyl sulfate (SDS) / sodium carboxyméthylcellulose (NaCMC), gélatine / SDS / NaCMC, hydrolysat de collagène / chitosan, pectine / béta-lactoglobuline, gomme d'acacia / béta-lactoglobuline, gomme d'acacia / protéine de pois, CMC / protéine de pois, alginate / protéine de pois, alginate / albumine, gluten de blé / caséine.

L'homme du métier pourra encore se référer à l'enseignement des documents Kamdem Eugene Patrickab et al., « Microencapsulation by complex coacervation of fish oil using gelatin/SDS/NaCMC », PAK. J. FOOD SCI., 23(1), 2013: 17-25 ; ou Xiao Jun-xia et al, « Microencapsulation of sweet orange oil by complex coacervation with soybean protein isolatelgum Arabie », Food Chemistry 125 (2011) 1267-1272*.*

Les polyélectrolytes hydrosolubles formant la membrane de la microcapsule peuvent être dans un état non-réticulé ou dans un état réticulé.

Par « réticulation », on entend la formation d'un réseau tridimensionnel, par voie chimique ou physique, formés par des liaisons entre les polyélectrolytes hydrosolubles.

En effet, en raison de la nature ionique des interactions entre les polyélectrolytes de la membrane, les microcapsules formées par coacervation complexe sont potentiellement fragiles. La réticulation limite donc le risque d'éclatement des microcapsules.

A ce sujet, l'homme du métier pourra encore se référer au document Izabela Dutra ALVIM et al, « Microparticles obtained by complex coacervation: influence of the type of reticulation and the drying process on the release of the core material », Ciênc. Tecnol. Aliment., Campinas, 30(4): 1069-1076, out.-dez. 2010*.*

### Particules solides aptes à former une émulsion de Pickering

Les microcapsules de l'invention sont caractérisées en ce que le système d'enveloppe comprend également des particules solides choisies parmi les particules solides aptes à stabiliser (ou « à former » ou « à assurer l'obtention d' ») une émulsion dite de Pickering.

Par « émulsion de Pickering », on entend en particulier une dispersion de deux liquides non-miscibles, stabilisée par des particules solides. L'adsorption des particules à l'interface de deux phases est responsable de la stabilisation de cette émulsion de Pickering.

En l'espèce, le déposant démontre que la formation de microcapsules, partant d'une émulsion de Pickering, permet d'obtenir des microcapsules dont la stabilité ainsi que la résistance mécanique sont améliorées.

Par souci de simplification, ces particules solides sont désignées encore sous l'appellation de « particules solides de Pickering ».

De préférence, les particules solides de Pickering sont choisies parmi les particules solides organiques et/ou inorganiques.

Les particules organiques utilisables selon l'invention sont par exemple des particules solides dérivées de l'amidon, du chitosan, de la chitine ou de protéines.

De telles particules organiques sont avantageusement choisies parmi les protéines solubles (avantageusement les protéines végétales, notamment les protéines de pois) qui sont aptes à former, d'une part, des particules solides de Pickering et, d'autre part, l'un des polyélectrolytes hydrosolubles.

Les particules inorganiques utilisables selon l'invention peuvent être d'origine naturelle ou non.

Par exemple la particule inorganique peut être sélectionnée parmi le kaolin, l'argile colloïdale (comme la bentonite), la silice colloïdale, l'alumine, le calcaire, la bauxite, le gypse, le carbonate de magnésium, le carbonate de calcium (sol et / ou précipité), la perlite, la dolomite, la diatomite, l'huntite, la magnésite, la boehmite, la palygorskite, le mica, la vermiculite, l'hydrotalcite, l'hectorite, l'hallyosite, la gibbsite, la kaolinite, la montmorillonite, l'illite, l'attapulgite, la laponite, le latex, et sépiolite, ou leur combinaison.

Les particules inorganiques choisies parmi l'argile colloïdale, la silice colloïdale, et le kaolin et leurs mélanges, sont particulièrement avantageux.

De manière générale, les particules ont avantageusement une taille comprise entre 1µm et 20 µm.

Ces particules sont utilisées avantageusement avec une concentration comprise entre 1 % et 5 %, en poids par rapport au poids des microcapsules.

Dans certains modes de réalisation, les particules solides sont sous forme d'un mélange de différents types de particules organiques et/ou inorganiques.

Par exemple, il est possible d'utiliser des mélanges - de particules inorganiques, comme la silice colloïdale ou l'argile (colloïdale ou non) avec - des particules organiques, notamment dérivées du chitosan, de l'amidon, de la gomme xanthane, de la chitine ou de protéines.

Les particules solides de Pickering, ainsi que la formation de telles émulsions de Pickering, sont encore détaillées dans le document Chevalier at al., « Emulsions stabilized with solid nanoparticles : Pickering emulsions », Colloids and Surfaces A : Physicochem. Eng. Aspects 439 (2013) 23-34*.*

### Tensioactifs

L'utilisation de particules solides pour stabiliser l'émulsion, préalablement à la coacervation complexe, permet de s'affranchir de l'utilisation de tensioactifs classiques.

Ainsi, dans certains modes de réalisation, les microcapsules de l'invention sont dépourvues de tensioactifs, notamment de tensioactifs synthétiques ou chimiques.

Un tel mode de réalisation est particulièrement avantageux dans les domaines de la santé, de la nutrition ou de la cosmétique.

### Contenu hydrophobe des microcapsules

Le contenu hydrophobe de la microcapsule (en général un liquide hydrophobe) peut être constitué d'un liquide pur, d'un mélange de liquides miscibles, d'une ou plusieurs matières solides dissoutes dans un liquide, d'une émulsion d'un liquide hydrophile ou encore d'une solution dans un liquide hydrophobe.

Typiquement, le contenu hydrophobe de la microcapsule selon l'invention est choisi parmi - les huiles (notamment les huiles végétales ou les huiles pharmaceutiques), - les solvants organiques ou - les matériaux à changement de phase.

Toute huile végétale peut être utilisée selon la présente invention. L'homme du métier pourra cependant adapter le choix de l'huile végétale en fonction du domaine d'utilisation des microcapsules et du produit à encapsuler.

A titre d'exemple, l'huile végétale peut être choisie parmi les huiles de colza, de palme, d'olive, de tournesol, de coco, de coprah, de noix, de lin, de bourrache, de sésame, d'argan, d'arachide, de cameline, de pépins de raisins, de pépins de courges, etc., ainsi que les mélanges d'au moins deux desdites huiles végétales.

Les huiles pharmaceutiques sont adaptées à un usage pharmaceutique, cosmétique ou agroalimentaire. Il s'agit généralement - de dérivés raffinés d'huiles végétales (comme le miglyol^{®}, qui est un dérivé de l'huile de coco ou de coprah), - d'huiles de poisson, ou - d'huiles minérales (tels que la paraffine et ses dérivés ou les huiles blanches pharmaceutiques).

On entend par « matériau à changement de phase », un matériau capable de changer d'état physique dans une plage de température restreinte. Cette plage est généralement située entre 10 et 80°C.

Dans cet intervalle de température, le changement de phase prépondérant reste la fusion/solidification. De tels matériaux peuvent être des composés inorganiques, comme des sels hydratés ou des composés organiques, comme le phénol.

Le contenu hydrophobe peut encore consister en une poudre hydrophobe ou une cire, ayant des propriétés hydrophobes.

### Principe actif

Le contenu hydrophobe contient également au moins un principe actif.

Ce principe actif peut constituer, avantageusement, de 1 % à 100 % du contenu hydrophobe précité.

Il peut s'agir d'un principe actif pharmaceutique, cosmétique, d'additifs alimentaires, de phéromones, de produits phytosanitaires, de cellules, de microorganismes ou encore de catalyseurs de réactions chimiques.

Dans le domaine alimentaire en particulier, un principe actif peut être choisi, par exemple, parmi les arômes, les minéraux, les probiotiques, les vitamines, les acides gras, les antioxydants, etc.

De tels principes actifs sont décrits mais ils ne font pas partie de l'invention. Le principe actif consiste en un composé chimique qui est sensible aux rayonnements ultraviolets (UV), dit encore « composé photosensible ».

Par « rayonnements ultraviolets », on entend les ondes électromagnétiques situées entre la lumière visible et les rayons X.

Ces rayonnements englobent quatre domaines : UV-A2 (340 nm-400 nm), UV-A1 (315 nm-340 nm), UV-B (280 nm-315 nm) et UV-C (100 nm-280 nm).

Par « composé photosensible », on entend un composé présentant une perte d'activité lorsqu'il est soumis à des rayonnements UV ; cette perte d'activité est par exemple inférieure à 20%, voire comprise entre 20 % et 30 %, voire supérieure à 30%.

En effet, comme démontré dans la partie Exemple, la photostabilité d'un principe actif photosensible est augmentée lorsque ce principe actif est encapsulé dans les microcapsules selon l'invention. En d'autres termes, la perte d'activité est réduite pour le principe actif encapsulé par rapport au principe actif non encapsulé.

Les principes actifs photosensibles englobent par exemple les composés suivants : les insecticides (par exemple les pyréthrines), les produits répulsifs (par exemple géraniol), les fongicides, les herbicides, les bio-pesticides, les filtres solaires (par exemple avobenzone), les parfums, les vitamines hydrophobes (par exemple la vitamine A).

### Paramètre physicochimique des microcapsules

Une microcapsule selon l'invention a de préférence une taille inférieure à 800 µm, notamment allant de 1 à 100 µm.

De façon particulièrement préférée, une microcapsule de l'invention a une taille allant de 1 à 50 µm.

Cette taille peut être mesurée par granulométrie laser (voir par exemple Renliang Xu ; « Light scattering : A review of particle characterization applications » ; Particuology 18 (2015) 11-21 ou les normes ISO 13320 :2009 et ISO 21501-2 :2007).

Sans être limité par une quelconque théorie, la taille et la stabilité des microcapsules sont influencées notamment par la concentration en polyélectrolytes hydrosolubles (c'est-à-dire en matériel de la membrane) et par la concentration en particules solides de Pickering.

Avantageusement, une concentration en biopolymères allant de 1 % à 15 % en poids par rapport au poids total des microcapsules est utilisée.

Le ratio protéine/polysaccharide dans le système d'enveloppe varie de 1/1 à 3/1, et de préférence de l'ordre 1/1.

Le ratio entre le contenu hydrophobe et la membrane (les polyélectrolytes hydrosolubles, notamment les biopolymères) formant le système d'enveloppe varie de 1/2 à 1/6.

### Composition contenant les microcapsules, et utilisation

Les microcapsules selon l'invention sont aptes à être incorporées dans une composition en vue de leur utilisation.

Les microcapsules selon l'invention peuvent alors être mélangées avec tout ingrédient actif ou tout excipient bien connu de l'homme du métier dans les domaines pharmaceutique, vétérinaire, cosmétique, agroalimentaire, agriculture, phytosanitaire, chimique et biomédical.

La composition consiste ainsi avantageusement en une composition pharmaceutique, vétérinaire, cosmétique, agroalimentaire, chimique et biomédicale.

Une telle composition est avantageusement une composition destinée à être administrée par voie orale à un sujet.

Dans ce cas, les microcapsules selon l'invention ont avantageusement l'intérêt de permettre un relargage contrôlé d'un principe actif au sein du tractus digestif d'un sujet.

Dans une telle application, les polyélectrolytes hydrosolubles et les particules de Pickering sont de préférence des composés non toxiques.

L'un des polyélectrolytes hydrosolubles est avantageusement une protéine, de préférence choisie parmi les protéines du lactosérum ou les protéines végétales comme les protéines de soja ou les protéines de pois.

L'autre des polyélectrolytes hydrosolubles est avantageusement un polysaccharide, de préférence choisi parmi la pectine, la gomme d'acacia, l'alginate de sodium ou la carboxyméthylcellulose.

Si des tensioactifs sont utilisés en complément des particules solides de Pickering pour former l'émulsion, il s'agit de tensioactifs reconnus comme non toxique selon les normes en vigueur dans le domaine pharmaceutique ou alimentaire.

Sans être limité par une quelconque théorie, les microcapsules selon l'invention permettent alors un adressage dudit au moins un principe actif au sein du tractus digestif d'un sujet de manière, d'une part, à protéger ledit au moins un principe actif vis-à-vis de conditions du milieu gastrique (pH acide, enzymes) et, d'autre part, à libérer ledit au moins un principe actif dans le milieu intestinal (milieu neutre à basique, par exemple milieu intestinal contenant notamment des ions phosphates, hydrogénocarbonate, carbonates).

Une telle composition peut encore consister en une composition destinée à être déposée / appliquée sur une surface à traiter.

Dans ce cas, les microcapsules sont en particulier adaptées à la protection, à l'égard des rayonnements ultraviolets, d'un principe actif sensible audits rayonnements ultraviolets.

Sans être limité par une quelconque théorie, les microcapsules selon l'invention confèrent une protection aux principes actifs photosensibles, à l'égard des rayonnements ultraviolets.

Les applications envisagées sont alors principalement phytosanitaires (et plus particulièrement sur la protection des plantes) ou cosmétique.

Les microcapsules selon l'invention ont en plus l'intérêt d'être particulièrement stables dans le temps et à la température (par exemple à 105°C pendant 24 heures).

### Procédé d'obtention des microcapsules

La présente invention se rapporte également au procédé pour la fabrication des microcapsules selon l'invention.

En substance, ce procédé selon l'invention comprend deux opérations successives :
- l'obtention de gouttelettes stabilisées par lesdites particules solides choisies parmi les particules solides aptes à stabiliser une émulsion de Pickering, puis
- le coacervation complexe desdites gouttelettes, par lesdits au moins deux polyélectrolytes hydrosolubles choisis parmi les polyélectrolytes hydrosolubles aptes à générer une microencapsulation par coacervation complexe.

### Emulsion de Pickering

Préalablement à l'opération de coacervation complexe, une émulsion de Pickering est générée.

Cette émulsion de Pickering, de type huile dans eau (H/E), est formée par émulsification d'une solution lipidique dans une solution aqueuse.

La solution lipidique constitue la phase dispersée de l'émulsion, et est destinée à constituer le contenu hydrophobe des microcapsules.

La solution aqueuse contient les particules de Pickering précitées, et est destinée à former la phase continue de cette émulsion.

De préférence, dans l'émulsion de Pickering, la taille des gouttelettes de contenu hydrophobe à encapsuler est inférieure à 300 µm, notamment inférieure à 200 µm. Avantageusement, les gouttelettes ont une taille allant de 5 à 200 µm.

La taille des gouttes dépend notamment de l'agitation du mélange lors de l'étape d'émulsification. De préférence, l'émulsification est réalisée dans un dispositif du type rotor / stator.

De préférence également, le rapport huile/eau de l'émulsion varie de 20/80 à 45/55, notamment de 25/75 à 50/50.

Dans certains modes de réalisation, des agents tensioactifs peuvent être ajoutés pour optimiser la formation de l'émulsion de Pickering. Ces agents tensioactifs ont été décrits ci-dessus.

### Coacervation complexe

L'opération de coacervation complexe peut être mise en œuvre selon trois variantes, suivant que l'un au moins des deux polyélectrolytes hydrosolubles est ajouté soit directement dans la solution aqueuse utilisée pour former l'émulsion de Pickering soit après la formation de ladite émulsion de Pickering (les deux polyélectrolytes hydrosolubles sont avantageusement ajoutés en même temps).

Typiquement, et quel que soit le mode de réalisation du procédé, le phénomène de microencapsulation par coacervation complexe est obtenue du fait des charges opposées entre les polyélectrolytes hydrosolubles rapportés.

Ces polyélectrolytes hydrosolubles complémentaires, ainsi que leur combinaison par couples de charges opposées, ont été décrits précédemment.

De façon avantageuse, cette combinaison de polyélectrolytes comprend au moins une protéine et un polysaccharide qui ont des charges opposées au pH de coacervation.

Dans un premier mode de réalisation, la solution aqueuse contenant les particules de Pickering comprend également au moins un premier polyélectrolyte hydrosoluble tel que défini précédemment.

Avantageusement, selon ce premier mode de réalisation, le procédé comprend les étapes suivantes :
(a) le mélange entre, d'une part, une solution lipidique et, d'autre part, une solution aqueuse contenant des particules solides de Pickering et au moins un des deux polyélectrolytes hydrosolubles, pour obtenir une émulsion de Pickering,
(b) le mélange de ladite émulsion de Pickering issue de l'étape (a) avec au moins un second polyélectrolyte hydrosoluble, ledit au moins un second polyélectrolyte hydrosoluble étant avantageusement en solution aqueuse,
(c) la microencapsulation par coacervation complexe du mélange issu de l'étape (b).

Dans ce premier de mode réalisation, à l'étape (a), les particules solides de Pickering et le premier polyélectrolyte hydrosoluble sont avantageusement constitués par un même composé ayant les deux propriétés recherchées.

Selon un second mode de réalisation, le premier et le second polyélectrolyte hydrosolubles sont ajoutés dans l'émulsion de Pickering, après sa formation.

De préférence, dans ce second mode de réalisation, le procédé comprend les étapes suivantes :
(a) le mélange d'une solution lipidique et d'une solution aqueuse contenant les particules solides de Pickering, pour la formation d'une émulsion de Pickering,
(b) le mélange de ladite émulsion de Pickering issue de l'étape (a) et de ladite combinaison de polyélectrolytes hydrosolubles, et
(c) la microencapsulation par coacervation complexe du mélange issue de l'étape (b).

Au cours de l'étape de mélange (b), les polyélectrolytes hydrosolubles sont ajoutés de façon simultanée ou successive à ladite émulsion de Pickering issue de l'étape (a), avantageusement chacun sous forme d'une solution aqueuse (c. à d. dissous ou en suspension dans une solution aqueuse).

Avantageusement, l'étape b) du type « successive », comprend :
(b1) le mélange de ladite émulsion de Pickering issue de l'étape (a) avec une première solution aqueuse comprenant ledit premier polyélectrolyte hydrosoluble, avantageusement ledit premier polyélectrolyte cationique, pour obtenir un premier mélange,
(b2) le mélange dudit premier mélange issue de l'étape (b1) avec une seconde solution aqueuse contenant ledit second polyélectrolyte hydrosoluble de charge opposée, avantageusement ledit second polyélectrolyte anionique, pour obtenir un second mélange.

Dans un troisième mode de réalisation, la solution aqueuse contenant les particules de Pickering comprend également les premier et second polyélectrolytes hydrosolubles tels que définis précédemment.

Avantageusement, selon ce troisième mode de réalisation, le procédé comprend les étapes suivantes :
(a) le mélange entre, d'une part, une solution lipidique et, d'autre part, une solution aqueuse contenant des particules solides de Pickering et les deux polyélectrolytes hydrosolubles, pour obtenir une émulsion de Pickering,
(b) la microencapsulation par coacervation complexe du mélange issu de l'étape (a).

Sans être lié par une quelconque théorie, ce troisième mode de réalisation permet une meilleure maitrise du procédé (notamment sur le plan de la reproductivité et de la répétabilité) ; ce mode permet également de réduire les pertes liées au transvasement à chaque étape et de simplifier le procédé.

De manière générale, les polyélectrolytes hydrosolubles sont incorporés sous agitation, par exemple sous agitation magnétique.

De manière générale, le polyélectrolyte hydrosoluble cationique est avantageusement une protéine.

Dans ce cas, l'étape de coacervation complexe comprend avantageusement une modification du pH dudit mélange obtenu à l'issue de l'étape b) pour les premier et le second modes et de l'étape a) pour le troisième mode.

Le cas échéant, cette modification du pH consiste en une acidification depuis un pH supérieur au pl de la protéine dont la charge globale est alors négative, jusqu'à atteindre un pH de coacervation inférieur au pl de ladite protéine pour obtenir une charge globale positive.

De manière générale, plusieurs facteurs physico-chimiques influencent la formation du coacervat complexe, à savoir principalement le pH, la force ionique, le ratio entre les polyélectrolytes hydrosolubles de charges opposées (le cas échéant, le ratio protéine / polysaccharide) et la concentration en polyélectrolytes hydrosolubles.

L'homme du métier peut ajuster ces différents paramètres, en tenant compte notamment de ses connaissances générales.

Les interactions entre protéines et polysaccharides sont observées lorsque ces deux biopolymères sont de charges opposées, soit généralement lorsque le pH du mélange aqueux obtenu à l'issue de l'étape b) pour les premier et second modes, et de l'étape a) pour le troisième mode, est inférieur au pl de la protéine.

Typiquement ce pH est acidifié, de sorte que la protéine forme un polycation de charge opposée à celle du polysaccharide. L'association de ces biopolymères de charge opposée, principalement par des interactions électrostatiques, conduit à la formation de complexes insolubles qui forment alors des gouttelettes de liquides composées des polymères (les polyélectrolytes) et de molécules de solvant, le coacervat.

Une séparation de phase est ensuite observée si le pH est suffisamment abaissé par rapport au pl de la protéine, tout en veillant à contrôler cette baisse pour éviter une neutralisation du polysaccharide.

L'homme du métier peut alors aisément déterminer la gamme de pH dans laquelle un rendement optimal de coacervation complexe est obtenu en faisant varier le pH lors de l'étape de coacervation complexe, comme décrit dans l'étude de Liu S., et al., (2010) « Effect of pH on the functional behavior of pea protein isolate-gum Arabic complexes", Food Research International, 43: 489-495.

L'acidification peut être obtenue par l'ajout d'une solution acide dans le mélange, par exemple d'acide acétique.

La force ionique joue un rôle important dans la formation du coacervat, car elle affecte également la charge des polyélectrolytes. Ainsi une force ionique très faible ou très élevée entraîne une suppression des forces électrostatiques entre protéines et polysaccharides.

La force ionique est influencée par la concentration en sels du mélange obtenu à l'issue de l'étape de coacervation complexe (étape (c) pour les premier et second modes, et étape (b) pour le troisième mode).

La taille et la stabilité des microcapsules est encore fonction de différents paramètres comme la concentration en polyélectrolytes hydrosolubles, le ratio protéine/polysaccharide dans le système d'enveloppe ou encore le ratio entre le matériau encapsulé et le matériel formant le système d'enveloppe (les polyélectrolytes hydrosolubles, notamment les biopolymères). Des valeurs avantageuses de ces paramètres ont été décrites précédemment.

A l'issue de ces étapes, on obtient alors des microcapsules selon l'invention dont le système d'enveloppe combine - une membrane de coacervation complexe et - des particules solides de Pickering.

### Refroidissement

La coacervation complexe est avantageusement suivie d'une étape de refroidissement.

Cette étape de refroidissement permet la solidification de la membrane de polyélectrolytes hydrosolubles.

Lorsque l'un des biopolymères utilisé possède des propriétés gélifiantes (comme la gélatine), cette étape permet la rigidification / solidification du système d'enveloppe.

Alternativement, un gélifiant peut être ajouté dans le mélange préalablement à l'étape de coacervation complexe (étape (c) pour les premier et second modes et étape (b) pour le troisième mode).

De manière générale, l'étape de refroidissement est réalisée à une température inférieure à 15 °C, notamment à une température comprise entre -5 et +10 °C, de préférence entre -1 et +5°C.

L'étape de refroidissement peut éventuellement être réalisée à un pH alcalin (soit supérieur à 7), de préférence compris entre 8 et 10 pour participer à la rigidification. Généralement l'alcalinisation de la solution de microcapsules est obtenue par ajout d'un agent alcalin comme l'hydroxyde de sodium (NaOH).

### Réticulation

L'étape de coacervation complexe peut également être suivie d'une étape de réticulation desdits polyélectrolytes hydrosolubles par le biais d'au moins un agent réticulant.

Une polymérisation efficace peut être obtenue par un traitement avec un dialdéhyde, comme le glutaraldéhyde. Cependant, la toxicité de ces derniers limite leur usage.

De préférence, la réticulation est donc réalisée avec des agents réticulants non toxiques, tels que les enzymes (transglutaminases ou lactases), les tanins (comme l'acide tannique), un composé iridoïde (comme la génipine) ou encore les polyphénols notamment les composés bioflavonoïdes (par exemple la proanthocyanidine), un extrait de pépin de raisin, un phosphopeptide de caséine-phosphate de calcium amorphe.

De préférence, la génipine, un agent réticulant enzymatique comme la transglutaminase, ou encore l'acide tannique, est utilisé.

Les enzymes, comme la transglutaminase, sont de préférence utilisées à une concentration allant de 10 à 25 U /g de protéine (utilisée en tant que polymère cationique).

### RESULTATS EXPERIMENTAUX

Les exemples ci-après illustrent plusieurs modes de réalisation de microcapsules selon l'invention, sans pour autant limiter la portée de l'invention.

### 1. Exemple 1 - Produit A

### 1.1. Procédé d'obtention

**Tableau 1 : Procédé d'obtention des microcapsules A**

| Exemple 1 : microcapsules A | Masse (g) | pH | T°C |
|---|---|---|---|
| EMULSION PICKERING | | | |
| Solution A contenant les particules solides type silices colloïdales | 80 | 9,0 | 50 |
| Ajout la phase lipophile dans la solution A | 41 | 8,0 | 47 |

| Homogénéisation Ultra turrax | | | |
|---|---|---|---|
| COACERVATION | | | |
| Ajout de la solution des deux polymères gélatine et gomme arabique | 60 | | |

| Agitation pâle à 55°C | | | |
|---|---|---|---|
| ACIDIFICATION | | | |
| Ajout d'acide acétique | 3,82 | 4,0 | 48 |

| Agitation magnétique | | | |
|---|---|---|---|
| SOLIDIFICATION | | | |
| Refroidissement (bain de glace) sous agitation magnétique (≈ 30 minutes) | | | 10°C |

Après refroidissement, NaOH est ajouté dans la solution de microcapsules afin d'obtenir un pH alcalin.

La transglutaminase est ajoutée à une concentration comprise entre 5 et 20U/g de protéine, pour obtenir une réticulation de la membrane. La réaction se déroule toute la nuit à température ambiante (RT).

### 1.2. Propriétés des microcapsules

### 1.2.1. Stabilité des microcapsules et distribution en taille des microcapsules

Afin de vérifier la stabilité des microcapsules formées, la distribution en taille des microcapsules a été réalisée avant et après centrifugation pendant 5 min, à 722 g.

Comme on peut l'observer sur le tableau 2 ci-dessous, les microcapsules produites sont stables et leur taille varie entre 30 et 110 microns.

**Tableau 2 : Distribution en taille des microcapsules A avant et après centrifugation.**

| PSD (µm) | D(v, 0,1) | D(v, 0,5) | D(v, 0,9) | D[4, 3] | Span |
|---|---|---|---|---|---|
| Microcapsules avant centrifugation | 34,78 | 79,45 | 167,48 | 90,66 | 1,66 |
| Microcapsules après centrifugation | 33,92 | 93,81 | 212,89 | 109,10 | 1,86 |

Dans ce tableau :
- D (v; 0,1) = taille de particule pour laquelle 10% de l'échantillon se trouve en dessous de cette dimension ;
- D (v; 0,5) = taille de particule à laquelle 50% de l'échantillon a une taille inférieure et 50% de l'échantillon a une taille supérieure ;
- D (v; 0,9) = taille de particule pour laquelle 90% de l'échantillon se trouve en dessous de cette dimension ;
- D (4; 3) = diamètre moyen (volume) ;
- Span = mesure de la largeur de la distribution

### 1.2.2. Morphologie des microcapsules

L'observation au microscope des microcapsules, lorsqu'elles sont conservées à température ambiante, montre la présence d'une membrane (coacervat) de polymères.

En revanche, après un stockage à 105°C pendant 24H, la membrane est moins visible, voire inexistante.

### 2. Exemple 2 - Produit B

### 2.1. Procédé d'obtention

**Tableau 3 : Procédé d'obtention des microcapsules B**

| Exemple 2 : microcapsules B | Masse (g) | pH | T°C |
|---|---|---|---|
| EMULSION PICKERING | | | |
| Solution B contenant les particules solides type argile colloïdale | 80 | 9,0 | 50 |
| Ajout la phase lipophile dans la solution A | 40 | 8,0 | 47 |

| Homogénéisation Ultra turrax | | | |
|---|---|---|---|
| COACERVATION | | | |
| Ajout de la solution des deux polymères gélatine et gomme arabique | 60 | | 45 |

| Agitation pâle à 55°C | | | |
|---|---|---|---|
| ACIDIFICATION | | | |
| Ajout d'acide acétique | 1.70 | 4,0 | 48 |

| Agitation magnétique | | | |
|---|---|---|---|
| SOLIDIFICATION | | | |
| Refroidissement (bain de glace) sous agitation magnétique (≈ 30 minutes) | | | 10 |

Après refroidissement, le NaOH est ajouté dans la solution des microcapsules afin d'obtenir un pH alcalin. La transglutaminase est ajouté à une concentration comprise entre 10 et 20 U/g de protéine. La réaction se déroule toute la nuit à RT.

### 2.2. Propriétés des microcapsules

### 2.2.1. Distribution de la taille des microcapsules

Lorsque l'émulsion de Pickering est réalisée, la distribution en taille est déterminée (voir tableau 4).

Une augmentation de la taille des microcapsules est observée par rapport à la taille de l'émulsion.

Tout comme dans l'exemple 1, la taille des microcapsules avant et après centrifugation (5 min, à 722 g) a été mesurée.

Là encore, une très bonne stabilité des microcapsules est observée au vu des résultats granulométriques. Les tailles des microcapsules sont comprises entre 15 et 85 microns.

**Tableau 4 : Distribution en taille de l'émulsion et des microcapsules « B » avant et après centrifugation**

| PSD (µm) | D(v, 0,1) | D(v, 0,5) | D(v, 0,9) | D[4, 3] | Span |
|---|---|---|---|---|---|
| Microcapsu les avant centrifugation | 17,02 | 40,76 | 85,18 | 51,51 | 1,68 |
| Microcapsu les après centrifugation | 15,94 | 39,33 | 81,99 | 56,05 | 1,68 |

### 2.2.2. Morphologie des microcapsules

L'observation au microscope des microcapsules, lorsqu'elles sont conservées à température ambiante, montre la présence d'une membrane (coacervat) de polymères.

En revanche, après un stockage à 105°C pendant 24H, la membrane est moins visible, voire inexistante.

### 2.2.3. Stabilité des microcapsules à la température

L'incorporation des particules solides dans la formulation augmentent la stabilité des microcapsules à la température.

Lorsque des microcapsules sont formées par coacervation complexe (par exemple gélatine, gomme arabique), les microcapsules sont détruites après un stockage à 50°C pendant une semaine. En revanche l'utilisation de particules solides, en combinaison avec les polymères utilisés en coacervation complexe, montre une meilleure stabilité à 50°C. En effet les microcapsules obtenues par ce procédé sont complètement stables pendant cette même période de temps.

### 3. Relargage du paprika microencapsulé pour le produit B

Pour illustrer la libération du principe actif, du paprika (actif modèle) a été solubilisé dans le miglyol (utilisé comme solvant organique).

Ce mélange constitue le cœur de deux types de microcapsules :
- des microparticules témoins, formées par coacervation complexe entre la gélatine et la gomme arabique (sans particules solides), et
- des microparticules B selon l'Exemple 2 ci-dessus, conforme à l'invention.

Les figures 1 et 2 montrent le relargage du paprika (principe actif contenu dans la phase lipophile) dans des conditions particulières, à savoir dans un mélange d'éthanol et de miglyol (ratio éthanol / miglyol : 70/30).

Les figures 1 et 2 montrent clairement que, lorsque les microcapsules contiennent des particules solides (produit B), elles sont moins perméables que les microcapsules sans particules solides (témoin).

Même après un stockage à 50°C pendant une semaine (figure 2), la même tendance a été observée.

### 4. Exemple 3 - Produit C

### 4. 1. Procédé d'obtention

**Tableau 5 : Procédé d'obtention des microcapsules C**

| Exemple 3 : microcapsules C | Masse (g) | pH | T°C |
|---|---|---|---|
| EMULSION | | | |
| Solution A contenant les particules solides type silices colloïdales et au moins deux polymères | 60 | | 50 |
| Ajout la phase lipophile dans la solution A | 40 | | 47 |

| Homogénéisation Ultra turrax | | | |
|---|---|---|---|
| Agitation pâle à 55°C | | | |
| ACIDIFICATION | | | |
| Ajout d'acide acétique | 3,82 | 4,0 | 48 |

| Agitation magnétique | | | |
|---|---|---|---|
| SOLIDIFICATION | | | |
| Refroidissement (bain de glace) sous agitation magnétique (≈ 30 minutes) | | | 10°C |

### 4.2. Distribution de la taille des microcapsules

Les tailles des microcapsules sont comprises entre 5 et 40 microns.

**Tableau 6 : Distribution en taille des microcapsules « C » après rinçage**

| PSD (µm) | D(v, 0,1) | D(v, 0,5) | D(v, 0,9) | D[4, 3] | Span |
|---|---|---|---|---|---|
| Microcapsu les après rinçage | 6.0 | 19,5 | 38,0 | 26,0 | 1,7 |

### 5. Protection pour les produits photosensibles

Les microcapsules selon le produit C ont été utilisées pour l'encapsulation d'un filtre solaire sensible UV, à savoir l'avobenzone. Des essais d'irradiation par les ultraviolets ont été réalisés.

### Principe

L'étude a consisté à évaluer le maintien du niveau de protection des microcapsules développées contenant un filtre UV (avobenzone) après une irradiation contrôlée en UVA et UVB, par une méthode spectrophotométrique.

Le spectre global d'irradiation est équivalent du spectre solaire entre 290 nm et 400 nm. Il est obtenu par une source artificielle produite par un lampe xénon avec un filtre Shott WG320/1.5mm et un filtre UG5/2mm pour couper les radiations visible et infrarouges.

L'irradiation de la source est calculée pour délivrer de 1 à 4 « doses minimales erythémateuses » ou DEM pour un phototype 2 et pour une heure.

### Protocole

La solution de microcapsules est appliquée sur une ou plusieurs plaques de PMMA, et appliqué le temps nécessaire sous la lampe UV préalablement calibrée.

Un système de ventilation permet le refroidissement de l'échantillon pendant l'irradiation.

Pour calculer le pourcentage de photostabilté, la valeur initiale du SPF (« Sunburn Protection Factor » ou « FPS » pour « Facteur de Protection Solaire », ou encore plus simplement « IP » pour « Indice de Protection ») est d'abord calculé et ensuite le SPF après une irradiation de 60 min (550W/m2) est recalculé.

L'échelle de photostabilité définie est :
- perte d'activité > 30 %, le produit n'est pas photostable,
- perte d'activité comprise entre 20 et 30 %, le produit est photostable, et
- perte d'activité < à 20 %, le produit est très photostable.

Les résultats montrent que le filtre solaire est photostable mais cette photostabilité est significativement accrue avec les microcapsules selon l'invention : la photostabilité est de 80 % pour le produit C (filtre solaire encapsulé), contre une photostabilité de 50 % pour le filtre solaire non encapsulé.

## Revendications

1. Microcapsule comprenant un contenu hydrophobe entouré par un système d'enveloppe qui comprend une membrane comprenant une combinaison d'au moins deux polyélectrolytes hydrosolubles complémentaires choisis parmi les polyélectrolytes hydrosolubles aptes à générer une microencapsulation par coacervation complexe,
lequel contenu hydrophobe contient au moins un principe actif,
lequel au moins un principe actif est sensible aux rayonnements ultraviolets,
**caractérisée en ce que** ledit système d'enveloppe comprend également des particules solides choisies parmi les particules solides aptes à stabiliser une émulsion de Pickering.

2. Microcapsule selon la revendication 1, **caractérisée en ce que** les particules solides sont choisies parmi les particules inorganiques et/ou les particules organiques.

3. Microcapsule selon l'une quelconque des revendications 1 ou 2, **caractérisée en ce que** les polyélectrolytes hydrosolubles sont d'origine naturelle.

4. Microcapsule selon l'une quelconque des revendications 1 à 3, **caractérisée en ce que** ladite combinaison d'au moins deux polyélectrolytes hydrosolubles complémentaires comprend au moins deux polyélectrolytes hydrosolubles ayant des charges opposées, à savoir :
- au moins un premier polyélectrolyte hydrosoluble ayant des charges positives, de préférence une protéine, et
- au moins un second polyélectrolyte hydrosoluble ayant des charges négatives, de préférence un polysaccharide.

5. Microcapsule selon l'une quelconque des revendications 1 à 4, **caractérisée en ce que** les polyélectrolytes hydrosolubles sont dans un état réticulé.

6. Microcapsule selon l'une quelconque des revendications 1 à 5, **caractérisée en ce que** le contenu hydrophobe est choisi parmi les huiles, les solvants organiques ou un matériau à changement de phase.

7. Microcapsule selon l'une quelconque des revendications 1 à 6, **caractérisée en ce que** le système d'enveloppe est dépourvu d'agents tensioactifs synthétiques.

8. Microcapsule selon l'une quelconque des revendications 1 à 7, **caractérisée en ce que** ladite microcapsule a une taille inférieure à 800 µm, de préférence de 1 à 100 µm, de préférence encore de 1 à 50 µm.

9. Composition contenant des microcapsules selon l'une quelconque des revendications 1 à 8.

10. Procédé pour la fabrication de microcapsules selon l'une quelconque des revendications 1 à 8, **caractérisé en ce qu'**il comprend :
- l'obtention de gouttelettes stabilisées par des particules solides choisies parmi les particules solides aptes à stabiliser une émulsion de Pickering, puis
- la microencapsulation par coacervation complexe desdites gouttelettes par une combinaison d'au moins deux polyélectrolytes hydrosolubles complémentaires choisis parmi les polyélectrolytes hydrosolubles aptes à générer une microencapsulation par coacervation complexe.

11. Procédé pour la fabrication de microcapsules selon la revendication 10, **caractérisé en ce qu'**il comprend l'une des combinaisons d'étapes suivantes, à savoir :
- selon une première combinaison d'étapes :
(a) le mélange, d'une part, d'une solution lipidique et, d'autre part, d'une solution aqueuse contenant des particules solides, d'une part, aptes à stabiliser une émulsion de Pickering et, d'autre part, formant l'un desdits deux polyélectrolytes hydrosolubles, pour la formation d'une émulsion de Pickering,
(b) le mélange de ladite émulsion de Pickering issue de l'étape (a) avec au moins un second polyélectrolyte hydrosoluble complémentaire,
(c) la coacervation complexe du mélange issu de l'étape (b), pour obtenir lesdites microcapsules,
ou
- selon une seconde combinaison d'étapes :
(a) le mélange d'une solution lipidique et d'une solution aqueuse contenant des particules solides aptes à former une émulsion de Pickering, pour la formation d'une émulsion de Pickering,
(b) le mélange de ladite émulsion de Pickering issue de l'étape (a) et de ladite combinaison de polyélectrolytes hydrosolubles complémentaires, et
(c) la coacervation complexe de mélange issu de l'étape (b),
ou
- selon une troisième combinaison d'étapes :
(a) le mélange d'une solution lipidique et d'une solution aqueuse contenant des particules solides aptes à former une émulsion de Pickering et ladite combinaison de polyélectrolytes hydrosolubles complémentaires, pour la formation d'une émulsion de Pickering, et
(b) la coacervation complexe du mélange issu de l'étape (a).

12. Procédé pour la fabrication de microcapsules selon la revendication 11, **caractérisé en ce que** ladite étape de coacervation complexe comprend :
- la modification du pH du mélange, le cas échéant une acidification jusqu'à atteindre un pH inférieur au point isoélectrique d'une protéine formant l'un desdits polyélectrolytes hydrosolubles, et éventuellement
- un refroidissement dudit second mélange.

13. Procédé pour la fabrication de microcapsules selon l'une quelconque des revendications 10 à 12, **caractérisé en ce que** ladite étape de coacervation est suivie d'une étape de réticulation desdits polyélectrolytes hydrosolubles complémentaires.

14. Utilisation de microcapsules selon l'une quelconque des revendications 1 à 8, pour la protection, à l'égard des rayonnements ultraviolets, d'un principe actif sensible auxdits rayonnements ultraviolets.

## Patentansprüche

1. Mikrokapsel mit einem wasserabweisenden Inhalt, der von einem Hüllensystem umgeben ist, das eine Membran aufweist, die eine Kombination wenigstens zweier wasserlöslicher komplementärer Polyelektrolyte aufweist, die unter den wasserlöslichen Polyelektrolyten ausgewählt sind, die geeignet sind, eine Mikroverkapselung durch komplexe Koazervierung zu erzeugen,
wobei der wasserabweisende Inhalt wenigstens einen Wirkstoff enthält,
wobei wenigstens ein Wirkstoff für ultraviolette Strahlung empfindlich ist,
**dadurch gekennzeichnet, daß** das Hüllensystem auch feste Partikel aufweist, die unter den festen Partikeln ausgewählt sind, die geeignet sind, eine Pickering-Emulsion zu stabilisieren.

2. Mikrokapsel gemäß Anspruch 1, **dadurch gekennzeichnet, daß** die festen Partikel unter den anorganischen Partikeln und/oder den organischen Partikeln ausgewählt sind.

3. Mikrokapsel gemäß einem der Ansprüche 1 oder 2, **dadurch gekennzeichnet, daß** die wasserlöslichen Polyelektrolyte natürlichen Ursprungs sind.

4. Mikrokapsel gemäß einem der Ansprüche 1 bis 3, **dadurch gekennzeichnet, daß** die Kombination wenigstens zweier wasserlöslicher komplementärer Polyelektrolyte wenigstens zwei wasserlösliche Polyelektrolyte mit entgegengesetzten Ladungen aufweist, und zwar:
- wenigstens einen ersten wasserlöslichen Polyelektrolyten mit positiven Ladungen, vorzugsweise ein Protein, und
- wenigstens einen zweiten wasserlöslichen Polyelektrolyten mit negativen Ladungen, vorzugsweise ein Polysaccharid.

5. Mikrokapsel gemäß einem der Ansprüche 1 bis 4, **dadurch gekennzeichnet, daß** sich die wasserlöslichen Polyelektrolyte in einem vernetzten Zustand befinden.

6. Mikrokapsel gemäß der Ansprüche 1 bis 5, **dadurch gekennzeichnet, daß** der wasserabweisende Inhalt unter den Ölen, den organischen Lösungsmitteln oder einem Material mit Phasenänderung ausgewählt ist.

7. Mikrokapsel gemäß einem der Ansprüche 1 bis 6, **dadurch gekennzeichnet, daß** das Hüllensystem frei von sybthetischen oberflächenaktiven Mitteln ist.

8. Mikrokapsel gemäß einem der Ansprüche 1 bis 7, **dadurch gekennzeichnet, daß** die Mikrokapsel eine Größe von weniger als 800 µm, vorzugsweise von 1 bis 100 µm, besonders bevorzugt von 1 bis 50 µm aufweist.

9. Zusammensetzung, die Mikrokapseln gemäß einem der Ansprüche 1 bis 8 aufweist.

10. Verfahren zum Herstellen von Mikrokaseln gemäß einem der Ansprüche 1 bis 8, **dadurch gekennzeichnet, daß** es
- das Erhalten von Tröpfchen, die durch feste Partikel stabilisiert sind, die unter den zum Stabilisieren einer Pickering-Emulsion geeigneten festen Partikeln ausgewählt sind, dann
- die Mikroverkapselung durch komplexe Koazervierung der Tröpfchen durch eine Kombination wenigstens zweier wasserlöslicher kemplementärer Polyelektrolyte, die unter den wasserlöslichen Polyelektrolyten ausgewählt sind, die geeignet sind, eine Mikroverkapselung durch komplexe Koazervierung zu erzeugen,
aufweist.

11. Verfahren zum Herstellen von Mikrokapseln gemäß Anspruch 10, **dadurch gekennzeichnet, daß** es eine der folgenden Kombinationen von Schritten aufweist:
gemäß einer ersten Kombination von Schritten:
a) das Mischen einer fetthaltigen Lösung einerseits mit einer wäßrigen Lösung, die feste Partikel aufweist, die einerseits geeignet sind, eine Pickering-Emulsion zu stabilisieren und andererseits einen der beiden wasserlöslichen Polyelektrolyten bilden, um eine Pickering-Emulsion zu bilden, andererseits,
b) das Mischen der Pickering-Emulsion aus dem Schritt a) mit wenigstens einem zweiten wasserlöslichen komplementären Polyelektrolyten,
c) die komplexe Koazervierung der Mischung aus dem Schritt b) zum Erhalten der Mikrokapseln,
oder
gemäß einer zweiten Kombination von Schritten:
a) das Mischen einer fetthaltigen Lösung und einer wäßrigen Lösung, die feste Partikel aufweist, die geeignet sind, eine Pickering-Emulsion zu bilden, um eine Pickering-Emulsion zu bilden,
b) das Mischen der Pickering-Emulsion aus dem Schritt a) und der Kombination wasserlöslicher komplementärer Polyelektrolyte, und
c) die komplexe Koazervierung der Mischung aus dem Schritt b),
oder
gemäß einer dritten Kombination von Schritten:
a) das Mischen einer fetthaltigen Lösung und einer wäßrigen Lösung, die feste Partikel aufweist, die geeignet sind, eine Pickering-Emulsion zu bilden, und der Kombination der wasserlöslichen komplementären Polyelektrolyte, um eine Pickering-Emulsion zu bilden, und
b) die komplexe Koazervierung der Mischung aus dem Schritt a).

12. Verfahren zum Herstellen von Mikrokapseln gemäß Anspruch 11, **dadurch gekennzeichnet, daß** der Schritt der komplexen Koazervierung
- die Änderung des pH-Werts der Mischung, gegebenenfalls die Versauerung bis zum Erreichen eines pH-Wert unterhalb des isoelektrischen Punkts eines einen der wasserlöslichen Polyelektrolyten bildenden Proteins und gegebenenfalls
- eine Kühlung der zweiten Mischung
aufweist.

13. Verfahren zum Herstellen von Mikrokapseln gemäß einem der Ansprüche 10 bis 12, **dadurch gekennzeichnet, daß** auf den Schritt der Koazervierung ein Schritt der Vernetzung der wasserlöslichen komplementären Polyelektrolyte folgt.

14. Verwendung der Mikrokapseln gemäß einem der Ansprüche 1 bis 8 für den Schutz eines gegen ultraviolette Strahlen empfindlichen Wirkstoffs vor ultravioletten Strahlen.

## Claims

1. A microcapsule comprising a hydrophobic content surrounded by a shell system that comprises a membrane comprising a combination of at least two complementary water-soluble polyelectrolytes chosen from among the water-soluble polyelectrolytes capable of generating microencapsulation by complex coacervation,
said hydrophobic content containing at least one active principle,
said at least one active principle being sensitive to ultraviolet radiation,
**characterized in that** said shell system also comprises solid particles chosen from among the solid particles capable of stabilizing a Pickering emulsion.

2. The microcapsule according to claim 1, **characterized in that** the solid particles are chosen from among inorganic particles and/or organic particles.

3. The microcapsule according to any one of claims 1 or 2, **characterized in that** the water-soluble polyelectrolytes are of natural origin.

4. The microcapsule according to any one of claims 1 to 3, **characterized in that** said combination of at least two complementary water-soluble polyelectrolytes comprises at least two water-soluble polyelectrolytes having opposite charges, namely:
- at least one first water-soluble polyelectrolyte having positive charges, preferably a protein, and
- at least one second water-soluble polyelectrolyte having negative charges, preferably a polysaccharide.

5. The microcapsule according to any one of claims 1 to 4, **characterized in that** the water-soluble polyelectrolytes are in a crosslinked state.

6. The microcapsule according to any one of claims 1 to 5, **characterized in that** the hydrophobic content is chosen from among oils, organic solvents or a phase-change material.

7. The microcapsule according to any one of claims 1 to 6, **characterized in that** the shell system is free synthetic surfactants.

8. The microcapsule according to any one of claims 1 to 7, **characterized in that** said microcapsule has a size less than 800 µm, preferably from 1 to 100 µm, still preferably from 1 to 50 µm.

9. A composition containing microcapsules according to any one of claims 1 to 8.

10. A method for producing microcapsules according to any one of claims 1 to 8, **characterized in that** it comprises:
- obtaining droplets stabilized by solid particles chosen from among solid particles capable of stabilizing a Pickering emulsion, then
- microencapsulating said droplets by complex coacervation, with a combination of at least two complementary water-soluble polyelectrolytes chosen from among the water-soluble polyelectrolytes capable of generating a microencapsulation by complex coacervation.

11. The method for producing microcapsules according to claim 10, **characterized in that** it comprises one of the following combinations of steps, i.e.:
- according to a first combination of steps:
(a) mixing, on the one hand, a lipid solution and, on the other hand, an aqueous solution containing solid particles, on the one hand, capable of stabilizing a Pickering emulsion and, on the other hand, forming one of said two water-soluble polyelectrolytes, for forming a Pickering emulsion,
(b) mixing said Pickering emulsion from step (a) with at least one second complementary water-soluble polyelectrolyte,
(c) performing the complex coacervation of the mixture from step (b), to obtain said microcapsules,
or
- according to a second combination of steps:
(a) mixing a lipid solution and an aqueous solution containing solid particles adapted to form a Pickering emulsion, for forming a Pickering emulsion,
(b) mixing said Pickering emulsion from step (a) and said combination of complementary water-soluble polyelectrolytes, and
(c) performing the complex coacervation of the mixture from step (b),
or
- according to a third combination of steps:
(a) mixing a lipid solution and an aqueous solution containing solid particles adapted to form a Pickering emulsion and said combination of complementary water-soluble polyelectrolytes, for forming a Pickering emulsion, and
(b) performing the complex coacervation of the mixture from step (a).

12. The method for producing microcapsules according to claim 11, **characterized in that** said complex coacervation step comprises:
- modifying the mixture pH, as the case may be performing an acidification until reaching a pH lower than the isoelectric point of a protein forming one of said water-soluble polyelectrolytes, and potentially
- cooling said second mixture.

13. The method for producing microcapsules according to any one of claims 10 to 12, **characterized in that** said coacervation step is followed by a step of crosslinking said complementary water-soluble polyelectrolytes.

14. The use of microcapsules according to any one of claims 1 to 8, for protecting, against ultraviolet radiation, an active principle sensitive to said ultraviolet radiation.
